# EUROPÄISCHE PATENTSCHRIFT

(11) **EP 1 068 346 B1**
(45) Veröffentlichungstag und Bekanntmachung des Hinweises auf die Patenterteilung: **08.10.2003**
(21) Anmeldenummer: 99924673.9
(22) Anmeldetag: 18.03.1999
(51) Int. Cl.: C12P 19/30, C07H 19/10, C07H 19/20

(54) **NUCLEOTIDAKTIVIERTE DI- UND OLIGOSACCHARIDE SOWIE VERFAHREN ZU DEREN HERSTELLUNG**
NUCLEOTIDE ACTIVATED DISACCHARIDES AND OLIGOSACCHARIDES AND METHOD FOR THE PRODUCTION THEREOF
DISACCHARIDES ET OLIGOSACCHARIDES ACTIVES PAR DES NUCLEOTIDES ET LEUR PROCEDE DE PRODUCTION

(30) Priorität: 20.03.1998 DE 19812162
(43) Veröffentlichungstag der Anmeldung: 17.01.2001
(73) Patentinhaber: Forschungszentrum Jülich GmbH, 52425 Jülich (DE)
(72) Erfinder: ZERVOSEN, Astrid, B-4840 Welkenraedt (BE); ELLING, Lothar, D-52066 Aachen (DE); NIEDER, Veronika, D-52428 Jülich (DE); KAMERLING, Johannis, P., NL-3461 JE Linschoten (NL); VLIEGENTHART, Johannes, F., G., NL-3981 GX Bunnik (NL); GUTIERREZ GALLEGO, Ricardo, NL-3513 CN Utrecht (NL)
(86) Internationale Anmeldenummer: DE9900860
(87) Internationale Veröffentlichungsnummer: WO99047695

(56) Entgegenhaltungen:
- WO-A-94/29477
- HARTMANN, EVAMARIE; KOENIG, HELMUT: "Nucleotide-activated oligosaccharides are intermediates of the cell wall polysaccharide of Methanosarcina barkeri" BIOL. CHEM. HOPPE-SEYLER, Bd. 372, Nr. 11, 1991, Seiten 971-4, XP002112516
- DENAMUR, ROBERT; GAYE, PIERRE: "Isolation and identification of four UDP trisaccharides in the ewe mammary gland and colostrum" EUR. J. BIOCHEM., Bd. 19, Nr. 1, 1971, Seiten 23-35, XP002112517
- HASHIMOTO, HIRONOBU; ENDO, TSUYOSHI; KAJIHARA, YASUHIRO: "Synthesis of the First Tricomponent Bisubstrate Analog That Exhibits Potent Inhibition against GlcNAc:.beta.-1,4-Galactosyltransferase" J. ORG. CHEM., Bd. 62, Nr. 7, 1997, Seiten 1914-5, XP002112518
- SAUER, F. D.; BLACKWELL, B. A.; KRAMER, J. K. G.; MARSDEN, B. J.: "Isolation and characterization of the carbohydrate moiety bound to N-7-mercaptoheptanoyl-O-threonine phosphate" FEMS MICROBIOL. REV. , Bd. 87, 1990, Seiten 333-8, XP002112519
- HEEMSKERK, JOHAN W. M.; STORZ, THOMAS; SCHMIDT, RICHARD R.; HEINZ, ERNST: "Biosynthesis of digalactosyldiacylglycerol in plastids from 16:3 and 18:3 plants" PLANT PHYSIOL. , Bd. 93, Nr. 4, 1990, Seiten 1286-94, XP002112520

## Beschreibung

Die Erfindung betrifft die Herstellung von nucleotidaktivierten Di- und Oligosacchariden sowie *Nucleotidaktivierte Di- und Oligosaccharide*.

Glycanstrukturen auf Glycokonjugaten, wie z.B. Glycoproteine und Glycolipide, haben vielfältige biologische Funktionen in der intra- und intermolekularen Kommunikation, die in Übersichtsartikeln von Drickamer 1994 Molecular Glycobilogy Oxford University Press, Oxford; 53-87 ; Feizi 1991, Nature 314, 84-86; Hakomori 1989, Adv. Cancer Res. 52, 257-331; Hart 1992, Curr.Opin.Cell biol. 4, 1017-1023; Lasky 1995, Ann. Rev. Biochem. 64, 113-139. Lis und Sharon 1993, Eur.J. Biochem. 218, 1-27; Rademacher et al. 1988, Ann. Rev. Biochem. 57, 785-838; Varki 1993, Glycobiol. 3, 97-130; Varki 1994, Proc. Natl. Acad. Sci USA 91, 7390.7397 zusammengefaßt worden sind. Die bekanntesten Beispiele sind die Blutgruppenantigene AB0(H), die Lewis-Antigene (Le^{a,b,x,y}) die u.a. in sialylierter Form an der Zell-Zell-Adhäsion als Ligand von E-, P- und L-Selectin beteiligt sind sowie das Gal(α1-3)Gal-Epitop, das eine enorme immunologische Barriere bei der Xenotransplantation von tierischen Organen in den Menschen darstellt.

Die Biosynthese der Glycane von Glycoproteinen und Glycolipiden in Eukaryonten (z.B. Säugetiere) ist in einer Reihe von Übersichtsartikeln von Abeijon und Hirschberg 1992, TIBS 17, 31-36; Brockhausen 1995, New. Comp. Biochem Vol 29a, Elsevier Science, Amsterdam, 201-260, Hakomori 1986, Spektrum der Wissenschaften 90-100; Lehle and Tanner 1995, Glycoproteins, New Comp. Biochem Vol 29a Elsevier Science Amsterdam, 201-260; Verbert 1995 Glycoprotiens, New Comp. Biochem Vol 29a, Elsevier Science, Amsterdam, 145-152 ausführlich dokumentiert. In der N-Glycan-Biosynthese der eukayontischer Glycoproteine wird zunächst ein Dolicholdiphosphatoligosaccharid durch sequentiellen Transfer von Monosacchariden aus Nucleotid- oder Dolicholphosphat aktivierten Zukkern aufgebaut, das dann "en bloc" auf ein Protein übertragen wird. O-Glycane der Glycoproteine bzw. der Glycane der Glycosphingolipide werden durch sequentiellen Transfer von Monosacchariden direkt auf die Protein- bzw. Lipidstruktur aufgebaut. Als Substrate dienen hier nucleotidaktivierte Monosaccharide. An der Biosynthese der N- und O-Glycane sind Leloir-Glycosyltransferasen und im Fall der N-Glycane auch Glycosidasen beteiligt. Der Leloir-Biosyntheseweg zum Aufbau von glycosylierten Verbindungen ist in Syntheseweg 1 dargestellt.

Die Veröffentlichung im J. Org. Chem. 1997, 62, 1914-1915 offenbart einen nucleophilen Glycosyl-Acceptor (GlcNAcβ-Ome) für die β-1,4-Galactosyltransferase welcher diese Glycosyltransferase inhibiert.

In der Veröffentlichung Eur. J. Biochem. 19 (1971), 23 offenbart, daß Nucleotid aktivierte Di- und Oligosaccharide Substrate für β-Galactosidase sein können.

Die Veröffentlichung Biol. Chem. Hoppe-Seyler, 372, 971 (1991) postuliert einen Biosyntheseweg für nucleotidaktivierte Di- und Oligosaccharide in Methanosarcina barkeri

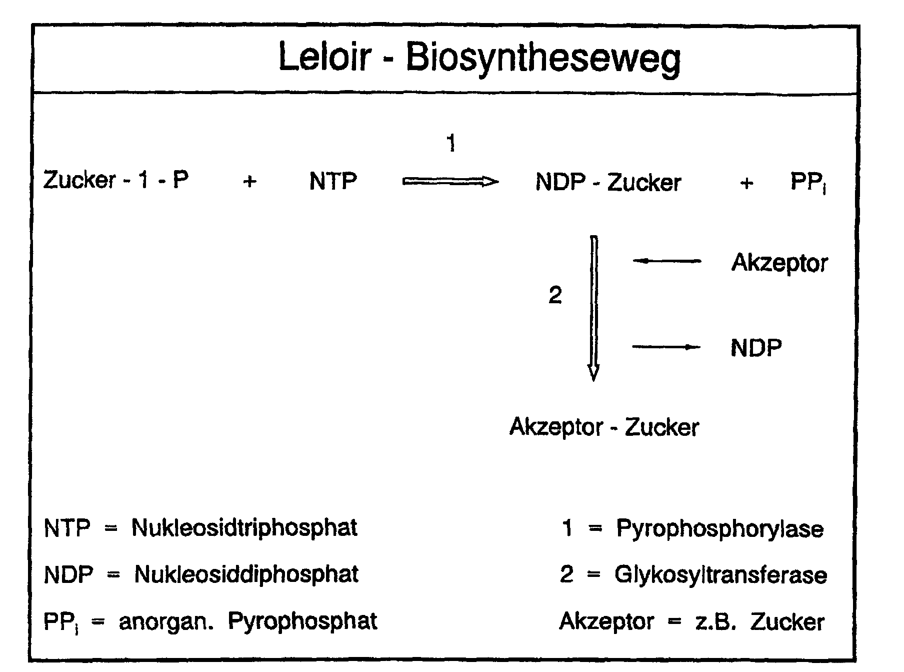

Syntheseweg **1**: Leloir-Biosyntheseweg für die Bildung von Nucleotidmonosacchariden und Übertragung durch Leloir-Glycosyltransferasen.

Gemäß Reaktionsschritt 1 katalysieren Pyrophosphorylasen die Synthese von nucleotidaktivierten Monosacchariden, indem sie einen Nucleotidylrest von einem Nucleosidtriphosphat auf einen am anomeren C-Atom phosphorylierten Zucker unter Freisetzung von Pyrophosphat übertragen. Die entstehenden Nucleotidmonosaccharide dienen im Reaktionsschritt 2 als Donorsubstrate von Leloir-Glycosyltransferasen, die den Zucker auf ein Akzeptorsubstrat unter Freisetzung von Nucleosiddiphosphat übertragen. Akzeptorsubstrate können Aglycone sein oder im Fall der Glycanbiosynthese Mono- oder Oligosaccharide. Bisher sind Nucleotidmonosaccharide als Akzeptorsubstrate von Leloir-Glycosyltransferasen noch nicht beschrieben bzw. auch nicht zur in vitro Synthese von nucleotidaktivierten Disacchariden eingesetzt worden.

In Arbeiten von Hindsgaul et al. 1995,J.Carbohydr. Chem. 14 453-464 sowie Srivastava et al. 1992, J. Biol. Chem. 267, 22356-22361 sind bereits modifizierte Trisaccharide chemisch hergestellt und als Donorsubstrate für die α1-3/4Fucosyltransferase aus Humanmilch eingesetzt worden. Dies wurde auch von Crawley und Palcic 1996 in Modern methods in carbohydrate synthesis (Frontiers in natural product research ser. Vol.1, Harwood Academ. Publichers, Amsterdam 492-517 berichtet. Es konnte dabei gezeigt werden, daß der Transfer von größeren Resten, wie z.B. Biotin oder die Trisaccharide der Blutgruppen A und B, auf LacNAc von einer GDP-aktivierten und C6-modifizierten Fucose möglich ist. In diesen chemisch hergestellten Fucosederivaten sind die größeren Reste über einen längeren Spacer an die Fucose gebunden.
Im Rahmen der Untersuchung des Substratspektrums von Fucosyltransferasen sowie der Entwicklung von möglichen Inhibitoren für diese Glycosyltransferasen wurden bereits auf chemischen Wege verschiedene nucleotidaktivierte Oligosaccharide synthetisiert (Rae und Turner 1996, XVIII Int. Carbohydr. Symp. Milano, BP 095 Streicher and Schmidt 1996, XVIII Ith Int. Carbohydr. Symp. Milano, BP038 sowie Tsuruta et al. 1996 XVIIIth Int. Carbohydr. Symp. Milano, BP084).

In Archaebakterien (z.B. Halobakterien) sind ebenfalls N- und O-Glycane (Thr-geknüpft) identifiziert worden. Als Intermediate ihrer prokaryontischen Biosynthese konnten nucleotidaktivierte Oligosaccharide isoliert und charakterisiert werden. Dies führte zur Formulierung eines hypothetischen Biosyntheseweges für die Biosynthese der Glycoproteine der Zellwand ("Surface" (S)-Layer) von *Methanothermus fervidus* (Hartmann et al. 1993, J. Bacteriol. 175, 4515-4519, sowie Hartmann and König 1989 Arch. Microbiol, 151, 274-281). Danach soll im Anschluß an die Phosphorylierung der Zucker zum Zukker-1-Phosphat die Bildung von nucleotidaktivierten Monosacchariden erfolgen, die dann zu nucleotidaktivierten Oligosacchariden verknüpft und anschließend 'en bloc' auf einen Lipidcarrier übertragen werden. Die beteiligten Enzyme wurden bisher noch nicht identifiziert.

Es ist die Aufgabe der Erfindung neuartige Glycanstrukturen sowie ein Verfahren zu deren Herstellung zu schaffen, die neue Anwendungen ermöglichen. Die Glycanstrukturen sollen als biochemische Reagenzien verwendbar sein.

Erfindungsgemäß wird die Aufgabe dadurch gelöst, daß Nucleotidaktivierte Di- oder Oligosaccharide mit n Saccharideinheiten hergestellt werden, indem Nucleotidsaccharide mit n-1 Saccharid-Einheiten als Akzeptoren mit Nucleosiddiphosphatzuckern (NDP-Zuckern) als Donatoren enzymatisch umgesetzt werden. Vorzugsweise erfolgt die Umsetzung mit einer Glycosyltransferase. Unter einem Oligosaccharid im Sinne der Erfindung ist ein Saccharid mit n Einheiten zu verstehen, wobei n mindestens 2, aber auch 3,4,5,6,7,8,9,10 ist und nach oberen Werten eine Anzahl von beipsielsweise 20 umfaßt. Jedoch soll nicht zwingend eine Begrenzung auf 20 bestehen, vielmehr soll eine frei wählbare Anzahl von Saccharideinheiten umfaßt werden, die der Fachmann noch nicht unter dem Begriff Polysaccharid versteht. Jedenfalls soll die Anzahl n nicht auf den für die Bezeichnung oligo typischen Bereich von n = 3 oder 10 Einheiten eingeschränkt sein.
Erfindungsgemäß können alle Pyrimidin- und Purinnucleotid-Saccharide mit n-1 Saccharid-Einheiten als Akzetoren eingesetzt werden. Dazu zählen Nucleotidsaccharide vom UDP-, ADP-, CDP-, DTDP-, GDP-,CMP- und IDP-Typ sowie deren Derivate, wie beispielsweise Azido-Derivate.

Beispielhaft sind weitere erfindungsgemäß einsetzbare Nucleotidsaccharide für Galactosyltransferasen Derivate des N-acetylglucosamins bzw. des Glucosamins. Als Donor-Verbindung können auch Derivate der UDP- Galactose, wie 2,3,4 oder 6 Desoxygalactose, Thiogalagtose, Galactosamin, sowie UDP- N-acetyl-Galactosamin, UDP-Glucose sowie UDP-Arabinose eingesetzt werden.

In einer bevorzugten Ausführungsform erfolgt die enzymatische Umsetzung mit einer eukaryontischen oder prokaryontischen Glycosyltransferase.

Weitere vorteilhafte Fortbildungen der Erfindung sind in den Unteransprüchen angegeben.

Das Nutzungspotential von nucleotidaktivierten Di- und Oligosacchariden als neuartige Verbindungen liegt in folgenden Bereichen;
1) Sie können als Standards in HPLC-Analysen von Zellextrakten dienen, um die Biosynthese nucleotidaktivierter Di- oder Oligosaccharide (z.B. UDP-LacNAc) nachzuweisen.
2) Nucleotidaktivierte Di- und Oligosaccharide können als Akzeptorsubstrate für weitere Leloir-Glycosyltransferasen oder Glycosidasen beim Aufbau von Glyco- und Neoglycokonjugaten dienen. Das am reduzierende Ende befindliche Nucleotid kann dabei als UV-Marker in der HPLC-Analytik oder zur Anbindung an einen Ionenaustauscher genutzt werden.
3) Ein sehr attraktiver Aspekt ist, nucleotidaktivierte Di- und Oligosaccharide als Donorsubstrate für Leloir-Glycosyltransferasen zu nutzen. Damit können ein enzymatischer 'en bloc' Transfer von Di- und Oligosacchariden auf einen Akzeptor *in vitro* etabliert werden.
4)Nucleotidaktivierte Di- und Oligosaccharide können auch als potentielle Inhibitoren von Leloir-Glycosyltransferasen und Nucleotidzuckertransportern eingesetzt werden.

Im Folgenden soll die Erfindung beispielhaft erläutert werden:

Mit der enzymatischen Synthese von Gal(β1-4)GlcNAc(α1-UDP mit verschiedenen eukaryontischen β1-4Galactosyltransferasen (β1-4GalT aus Rindermilch und Humanmilch sowie rekombinanter, humaner GalT, EC 2.4.1.38) konnte erfindungsgemäß erstmals gezeigt werden, daß eine Übertragung eines Monosaccharids von einem Nucleotidzucker, hier UDP-Gal, auf einen anderen Nucleotidzucker, hier UDP-GlcNAc, unter Bildung eines nucleotidaktivierten Disaccharids mit einer Leloir-Glycosyltransferase möglich ist (Syntheseweg 2). Erfindungegemäß kann im Gegensatz zum natürlichen Akzeptorsubstrat *N*-Acetyl-β-D-Glucosaminid (β-D-GlcNAc-OR, (R = z.B. Alkyl, Vinyl, Benzyl oder Monosaccharid) auch UDP-N-Acetyl-α-D-glucosamin als Akzeptorsubstrat fungieren (Syntheseweg 2).

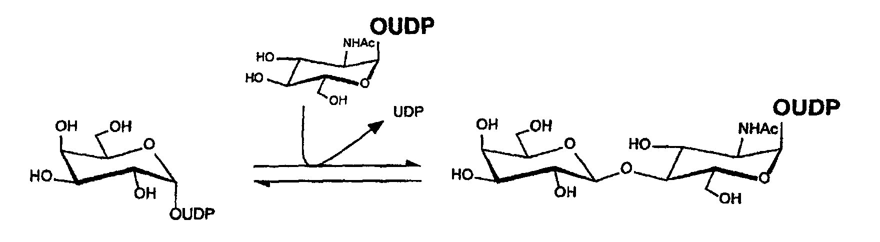

**Syntheseweg 2:** Synthese von Gal(β1-4)GlcNAc(α1-UDP mit β1-4Galactosyltransferase (EC 2.4.1.38).

Diese Reaktion wurde aufgrund der bekannten eukaryontischen Biosynthesewege sowie der bisherigen Untersuchungen zum Substratspektrum der β1-4GalT (β1-4Galactosyltransferase) nicht erwartet. Leloir-Glycosyltransferasen werden aufgrund einer gewissen Flexibilität für ihre Donor- und Akzeptorsubstrate zur Synthese von natürlichen und modifizierten Oligosacchariden eingesetzt. Die β1-4GalT katalysiert im allgemeinen den Transfer von D-Galactose aus UDP-Gal auf die 4-OH-Gruppe eines β-geknüpften N-Acetylglucosamins (β-D-GlcNAc-OR) und bildet eine N-Acetyllactosaminideinheit, wie von Baisch et al. Bioorgan. Med. Chem. Lett.1996, 6, 749-754; Crawley and Palcic, "Modern Methods in Carbohydrate Synthesis, Harwood Academic Publishers, Amsterdam, 1996, 492-517; David et al.Adv. Carbohydr. Chem. Biochem. 1991, 49, 175-237; Palcic und Hindsgaul, Trends in Glycoscience and Glycotrechnology 1996, 8, 37-49; Wong et al. J. Am. Chem. Soc. 1991, 113, 8137-8145; Wong und Whitesides "Enzymes in Synthetic Organic Chemistry, Elsevier Science, Oxford,1994, 252-297 gezeigt wurde. Inzwischen wurden die natürlichen Substrate UDP-Gal und β-D-GlcNAc-OR durch eine Vielzahl von Substratanaloga ersetzt die in der Veröffentlichung von Palcic und Hindsgaul, Trends in Glycosciences and Glycotechnology 1996, 8, 37-49 aufgelistet wurden. Diese als Substrate einsetzbaren Verbindungen (Donatoren) sowie deren nucleotidactivierte Formen (Akzeptoren) sollen alle von der Erfindung umfaßt und ausdrücklich zum Offenbarungsgehalt dieser Anmedlung gehören. Beispielhaft können die besonders wichtigen Substrate wie Derivate des N-acetylglucosamins oder des Glucosamins, 2,3,4 und 6 Desoxygalactose, Thiogalactose, Galactosamin, N-acetylgalactosamin, Glucose sowie Arabinose genannt werden . Jedoch stellen die genannten Verbindungen keine Beschränkung dar. Die Arbeitsgruppe Wong stellte fest, daß α-D-Glucosaminide von der β1-4GalT als Akzeptorsubstrate nicht gut umgesetzt werden (Takayama et al. Bioorgan. Med. Chem Lett. 1996, 6, 1123-1126). So sind alle bekannten Glycoside von GlcNAc, die Akzeptorsubstrate der Galactosyltransferase sind, β-glycosidisch verknüpft (Wong et al. Angew. Chem. 1995, 107, 569-593). Hingegen werden α- und β-Glucoside in Anwesenheit von α-Lactalbumin akzeptiert, wie von Wong et al. 1991 in Angew. Chem. 107, 569-593 gezeigt wurde. Aufgrund dieser Erkenntnisse wurde eine Umsetzung von UDP-Gal mit UDP-α-D-GlcNAc als Akzeptor nicht erwartet. Die Syntheseergebnisse zeigen jedoch, daß unter geeigneten Synthesebedingungen gute Ausbeuten an Gal(β1-4)GlcNAc(α1-UDP (Beispiele 1- 4) erzielt werden können. Das aktivierte Disaccharid Gal(β1-4)GlcNAc(α1-UDP ist beispielhaft für die erfindungsgemäßen enzymatisch hergestellten nucleotidaktivierten Disaccharide.

Überraschenderweise wurde festgestellt, daß bei tiefen Temperaturen, wie -20°C die in Syntheseweg 2 dargestellte Reaktion zu guten Ausbeuten führt, die sogar gegenüber einer Reaktonsführung von ca. 30°C erhöht sind. Die Glycosylsyltransferasen zeigen bei Temperaturen zwischen -10°C und -40°C eine erhöhte Enzymaktivität. Die Beispiele 3 und 4 zeigen, daß β1-4Galactosyltransferasen bei -20°C aktiv sind und daß die Ausbeuten der Synthesen im Vergleich zu denen bei 30°C bei gleicher Inkubationszeit gesteigert werden konnten. Bei den erfindungegemäßen Synthesen mit Leloir-Glycosyltransferasen im gefrorenem Reaktionsansatz werden Probleme der Sterilhaltung der Proben bei Synthesen mit langen Inkubationszeiten vermieden.

Das erfindungsgemäße nucleotidaktivierte Di- oder Oligosaccharid wird je nach verwendeter Glycosyltransferase Verknüpfungen der Saccharideinheiten an verschiedenen Positionen eines jeweiligen Zuckers aufweisen, diese sind dem Fachmann jedoch bekannt. Beispielhaft können L-Fucose, N-Acetylneuraminsäure, D-Galactose, D-xylose, D-Glucuronsäure, D-Galacturonsäure, D-Mannose, D-Glucose, N-Acetylglucosamin sowie N-Acetylgalactosamin genannt werden. Mit den erfindungsgemäßen Verfahren können NDP-N-acetyllactosamin sowie dessen Derivate oder eine NDP-Lactose sowie deren Derivate hergestellt werden. Durch eine Variation der während der Reaktion eingesetzten Donor-NDP-Zuckern können im Produkt Saccharidketten gebildet werden, die in ihrer Saccharid-Sequenz wählbar sind.

### Anwendungsbeispiele

Die im Folgenden beschriebenen Methoden wurden in allen Beispielen angewendet:

### Material und Methoden für die MS und NMR Analysen

### Massenspektrometrie

Die Molmassen der Reaktionsprodukte wurden mittels Fast Atom Bombardment Mass Spectrometry (FAB MS) im Negativ Ionen Modus an einem JEOL JMS-SX/SX 102A Viersektoreninstrument bei einer Beschleunigungsspannung von 6 keV durchgeführt. Das Gerät war mit einer JEOL MS-FAB 10D FAB gun ausgerüstet, die mit einem 10 mA Emissionstrom arbeitet und einen Strahl von 4 keV Xenon Atome erzeugt. 1.0 µl Probe (10µg in 0,1 ml 5% Essigsäure) wurde gelöst, in 1,0 µl Glycerin oder Thioglycerin Matrix dispergiert und lineare Massenabtastungen über 1000 Dalton aufgezeichnet. Die aufgezeichneten Daten wurden mit der JEOL Komplement Software ausgewertet (Bijvoet Center, Department of Mass Spectrometry).

### NMR Spektroskopie

Die Reaktionsprodukte wurden vor der Analyse wiederholt in D₂O (99,9 Atom % D; Isotec) aufgenommen und gefriergetrocknet und schließlich in 450 µl D₂O (99,96 Atom %D, Isotec) gelöst. Proton-entkoppelte 75.469-MHz ¹³C NMR Spektren wurden an einem Bruker AC-300 Spektrometer bei Probentemperaturen von 300 K aufgezeichnet. Chemische Verschiebungen (δ) beziehen sich auf externes Aceton (δ 31,08). Auflösungsverstärkte ¹H 1D and 2D NMR Spektren wurden an Bruker AMX-500 oder Bruker AMX-600 Instrumenten (Department of NMR spectroscopy, Utrecht University) bei Probentemperaturen von 300 K aufgenommen. Chemische Verschiebungen (δ) wurden in ppm bezogen auf internes Acetat (δ 1,908) ausgedrückt. HOD Signalunterdrückung wurde durch Anwendung einer WEFT Pulssequenz [ K. Hard, G. van Zadelhoff, P. Moonen, J.P. Kamerling, and J.F.G. Vliegenthart, Eur. J. Biochem., 209 (1992) 895-915. ]R. Koplin, J.-R. Brisson, and C. Whitfield, J. Biol. Chem., 272 (1997) 4121-4128] in 1D ¹H Experimenten und durch Vorsättigung für 1 s in 2D Experimenten erreicht.
2D TOCSY Spektren wurden mit Hilfe von MLEV-17 Mischungssequenzen mit effektiven Spin-Lock Zeiten zwischen 20 und 100 ms aufgezeichnet. 2D ROESY Spektren wurden mit einer Mischungszeit von 250 ms aufgezeichnet. Die Spin-Lock Feldstärke entsprach einem 90° Puls von ca. 115 µs. Proton ermittelnde ¹³C-¹H 2D HMBC Experimente wurden bei einer ¹H Frequenz von 500,139 und 600,140 MHz (125,769 und 150,916 MHz für ¹³C) mit einer von Summers et al. [M.F. Summers, L.G. Marzilli, and A. Bax, J. Am. Chem. Soc., 108 (1986) 4285-4294.] beschriebenen Pulssequenz durchgeführt. Die Verzögerungszeit für die Ermittlung der long-range ¹³C-¹H Kopplungen wurde auf 60 ms festgesetzt. ¹H 1D und 2D Spektren wurden an Silicon Graphics IRIS Workstationen (Indigo 2 and 02)mit Bruker UXNMR Software (Bijvoet Center, Department of NMR spectroscopy) ausgewertet. ¹³C 1D Spektren wurden an Silicon Graphics IRIS Workstationen (Indigo 2 and O2) mit TRITON Software (Bijvoet Center, Department of NMR spectroscopy) bearbeitet.

Den Anwendungsbespielen sind folgende Figuren zugehörig:
Figur 1: FAB MS (Negativ Ionen Modus, Thioglycerin) des UDP-LacNAc's, das mit β1-4 Galactosyltransferase aus Humanmilch synthetisiert wurde.
Figur 2: Struktur von Gal(β1-4)Glc[NAc](α1-UDP
Figur 3: 1D ¹H 500-MHz NMR Spektrum des UDP-LacNAc's, das mit β1-4 Galactosyltransferase aus Humanmilch synthetisiert wurde, bei 300K.
Figur 4: 1D ¹³C 75-MHz NMR Spektrum des UDP-LacNAc's, das mit β1-4 Galactosyltransferase aus Humanmilch synthetisiert wurde, bei 300K.
Figuren 5a). 5b) und 5c):
   HPLC-Chromatogramme der Gal(β1-4)GlcNAc(α1-UDP (UDP-LacNAc) Synthese mit verschiedenen β1-4Galactosyltransferasen.

### 1. Beispiel:

### Synthese von Gal(β1-4)GlcNAc(α1-UDP (UDP-LacNAc) mit β1-4Galactosyltransferase (EC 2.4.1.38) aus Humanmilch

Zur präparativen Synthese wurden 100 µmol (61 mg) UDP-Gal, 100 µmol (65.1 mg) UDP-GlcNAc (beides als Na₂-Salze von Sigma, Deisenhofen), 10 mg BSA, 4 U β1-4Galactosyltransferase aus Humanmilch in 10 ml 200 mM HEPES-NaOH pH 7.0 mit 1 mM MnCl₂ gelöst und unter sterilen Bedingungen inkubiert. Das Enzym wurde von Prof. Berger (ETH Zürich) zur Verfügung gestellt. Nach einer Inkubationszeit von 7 Tagen bei 30°C wurde die Reaktion durch ein kurzzeitige Hitzedenaturierung des Enzyms (5 min. auf 95°C) abgestoppt und mittels Ionenpaar-Reverse-Phase-HPLC Elling und Kula 1993 J. Biotechnology 29, 277-286 analysiert. Die Syntheseausbeute für UDP-LacNAc betrug 14.6% (bezogen auf den Akzeptor).

Die Produktisolierung erfolgte in 4 Schritten:
1) Abtrennung vorhandener Proteine durch Ultrafiltration in einer 10 ml Ultrafiltrationszelle von Amicon (Witten) mit einer YM 10-Membran (Ausschlußgröße: 10.000 Da).
2) Isolierung des Produktes UDP-LacNAc durch Gelfiltration mit Biogel P2 extra fine Material von Biorad (München) bei 4 °C und Abtrennung von UDP, UDP-GlcNAc und UDP-Gal: Die Anlage bestand aus einer Säule (Innendurchmesser: 2.6 cm; Betthöhe: 74.5 cm; Gelvolumen: 395 ml) und aus einer Pumpe P-1 von Pharmacia (Freiburg). Die Elution erfolgte mit Millipore Wasser mit einer Flußgeschwindigkeit von 0.25 ml/min. Es wurden 3 ml Fraktionen gesammelt und mit der HPLC-Analytik Elling und Kula 1993 J. Biotechnology 29, 277-286 analysiert.
3) Abtrennung des noch vorhandenen Puffers (HEPES) durch Anionenaustauschchromatographie: Die Chromatographieanlage bestand aus einer Säule mit 8 ml Sepharose Q FF, einer P1-Pumpe, einem Detektor UV-1 (254 nm), einem Fraktionssammler Frac 300 und einem Schreiber REC 101. Alle Materialien waren von Pharmacia (Freiburg). Die Säule wurde zunächst mit einem dreifachen Säulenvolumen dest. Wasser equilibriert. Anschließend wurde die Probe, deren pH-Wert auf 6.2 eingestellt worden war, aufgegeben. Die Säule wurde dann mit 30 ml dest. Wasser gespült. Das Produkt wurde mit einem linearen Salzgradienten (75 ml dest. Wasser, 75 ml 1 M LiCl) eluiert. Die Flußrate betrug jeweils 1 ml/min. Die Fraktionen, die UDP-LacNAc enthielten (HPLC-Analytik, Elling und Kula 1993), wurden gesammelt und der Pool bei 35°C im Wasserstrahlvakuum aufkonzentriert.
4) Entsalzung des Produktes durch Gelfiltration mit Biogel P2 (siehe oben) bei 4 °C und anschließende Gefriertrocknung.

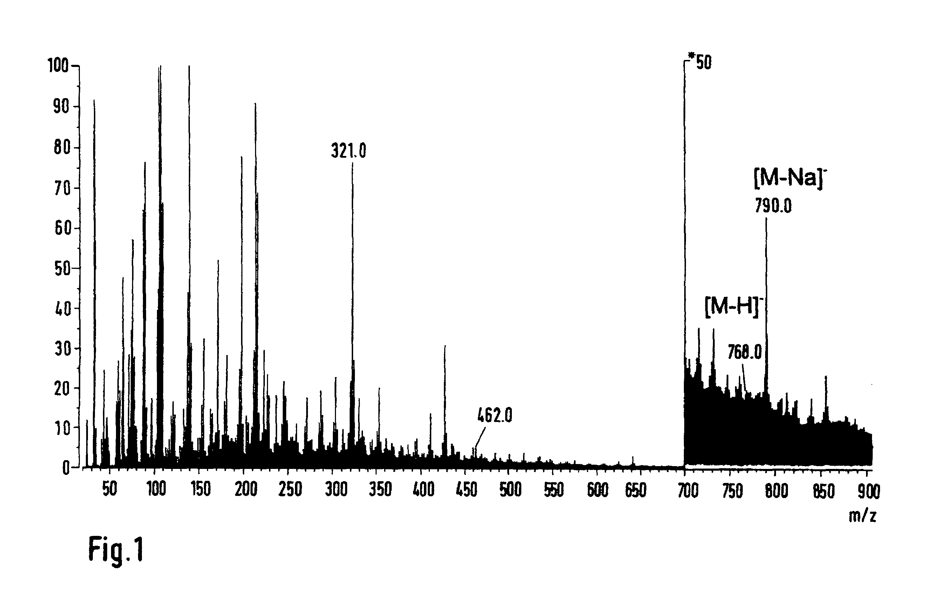

Insgesamt wurden 4.21 mg UDP-LacNAc isoliert (Gesamtausbeute: 5.2%).
Die Substanz ist mindestens 6 Monate bei -20°C stabil. UDP-LacNAc wurde durch NMR-Spektroskopie und Massenspektrometrie charakterisiert. (Fig.1-4)

Ergebnisse zur Galactosyltransferase:

Produkt Gal(β1-4)GlcNAc(α1-UDP synthetisiert mit β1-4GalT aus Humanmilch.

### Massenspektrometrie

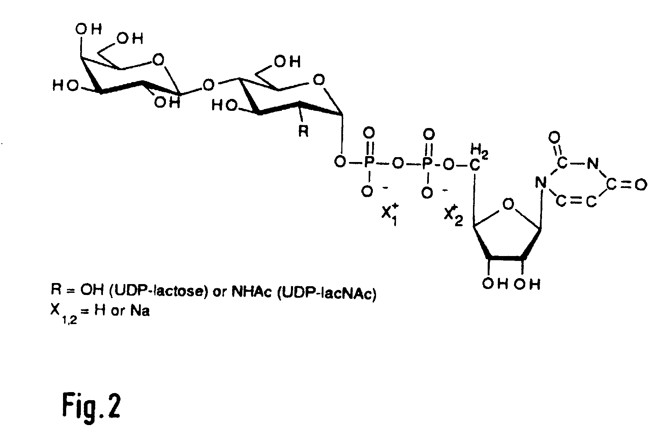

Das FAB MS (Negativ Ionen Modus, Thioglyccerin) Spektrum (Figur 1) des Reaktionsproduktes zeigte einen sehr starken Peak in der Hochmassenregion bei *m*/*z* 790,0, der dem [M-Na]⁻ pseudomolekularen Ion der Struktur (X_{1,2} = Na) in Figur 2 entspricht. Zusätzliche Fragmentionen werden bei *m*/*z* 462,0 und 321,0 gefunden.

### NMR Spektroskopie

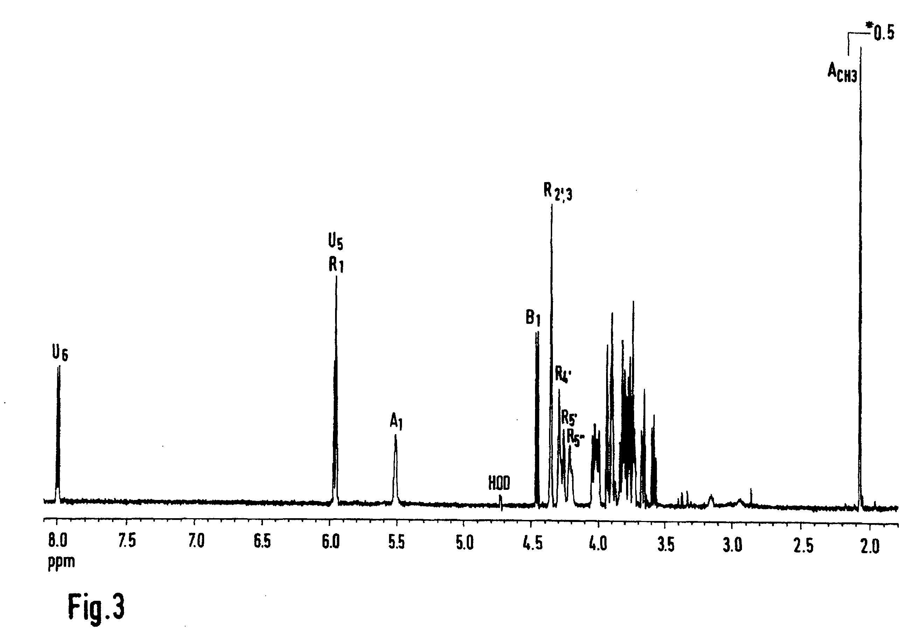

Das 1D ¹H NMR Spektrum (Figur 3) zeigte vier Signale im Tieffeld vom HOD Signal (δ4,751). Zwei dieser Signale, bei δ 8,002 (³*J*_{6,5} 8,1 Hz, U₆) and δ 5,969 (³*J*_{5,6} 8,1 Hz, U₅) wurden den Protonen des Uracilrings an Hand von Literaturdaten zugeordnet [ K. Hard, G. van Zadelhoff, P. Moonen, J.P. Kamerling, and J.F.G. Vliegenthart, Eur. J. Biochem., 209 (1992) 895-915. J.-H. Yoon and K. Ajisaka, Carbohydr. Res., 292 (1996) 153-163.]. Das anomere Signal bei δ 5,969 (³*J*_{1,2} 2,6 Hz) wurde dem Riboserest R (β Konfiguration, Furanoseringform) zugeordnet, wohingegen das verbleibende anomere Signal bei δ 5,511 (³*J*_{1,2} 3,2 Hz, ³*J*_{1,P} 7,2 Hz) dem GlcNAc-Rest A (α Konfiguration, Pyranoseringform), der mit der Phosphatgruppe verbunden ist, zugeordnet wurde. Zwei chemische Verschiebungen im Hochfeld von der HOD Resonanz konnten auch zugeordnet werden; das Singlet bei δ 2,072, das von den *N*-acetyl Protonen des GlcNAc's A stammt sowie das anomere Doublet bei δ 4,465 (³*J*_{1,2} 7,5 Hz), das den Gal-Rest B reflektiert (β Konfiguration , Pyranoseringform).

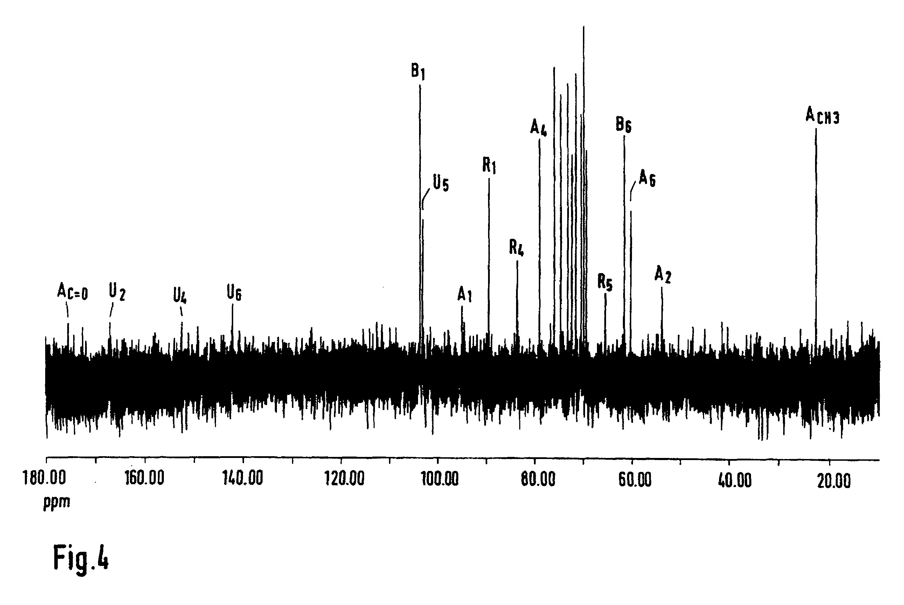

Das ¹³C NMR Spektrum (Figur 4) zeigte vier Signale in der anomeren Region bei δ 103,70 (B₁), 103,20 (U₅), 95,00 (A₁) und 89,80 (R₁). Weiterhin wurden vier Signale in der Tieffeldregion bei δ 175,55 (A_{C=O}), 166,70 (U₂), 152,30 (U₄) und 142,10 (U₆) beobachtet. Typische GlcNAc Resonanzen wurden bei δ 22,72 (NAc-CH₃) und d 53,89 (A₂) gefunden. Die Signale bei δ 60,30, 61,62 und 65,13 spiegeln die Anwesenheit von drei Hydroxymethylgruppen wider, von denen die letztere substituiert ist.

Durch 2D TOCSY, ROESY und HMBC Experimente konnten alle ¹³C und fast alle ¹H Resonanzen in den 1D Spektren zageordnet werden (Tabelle 1).

**Tabelle 1:**

| ¹H and ¹³C NMR chemische Verschiebungen des UDP-LacNAc's, das mit β1-4Galactosyltransferase aus Humanmilch synthetisiert wurde (aufgenommen bei 300 K). Die Kopplungskonstanten (Hz) sind in Klammern angegeben. | | |
|---|---|---|
| Rest | UDP-LacNAc ¹H^{a} | ¹³C^{b} |
| A-1 | 5.511 (3.2,7.2) | 95.00 |
| A-2 | 4.032 | 53.89 |
| A-3 | 3.806 | 70.29 |
| A-4 | 3.767 | 79.12 |
| A-5 | 3.998 | 72.37 |
| A-6a | n.d.^{c} | 60.30 |
| A-6b | 3.885^{c} | - |
| A CH₃ | 2.072 | 22.72 |
| A C=O | - | 175.5 |
| | | 5 |
| | | |
| B-1 | 4.465 | 103.7 |
| | (7.5) | 0 |
| B-2 | 3.573 | 71.66 |
| B-3 | 3.659 | 73.11 |
| B-4 | 3.933 | 68.97 |
| B-5 | 3.737 | 75.98 |
| B-6a | 3.872^{c} | 61.62 |
| B-6b | 3.792^{c} | - |
| | | |
| R-1 | 5.969 | 89.80 |
| | (2.6) | |
| R-2 | 4.364 | 74.76 |
| R-3 | 4.364 | 69.69 |
| R-4 | 4.289 | 83.93 |
| R-5a | 4.265 | 65.13 |
| R-5b | 4.212 | - |
| U-2 | - | 166.7 |
| | | 0 |
| U-4 | - | 152.3 |
| | | 0 |
| U-5 | 5.969 | 103.2 |
| | (8.1) | 0 |
| U-6 | 8.002 | 142.1 |
| | (8.1) | 0 |

| | | |
|---|---|---|
| ^{a} In ppm relativ zum Signal von internem Acetat. | | |
| ^{b} In ppm relativ zum Signal von externem Aceton. | | |
| ^{c} Die Zuordnung von H-6a und H-6b könnte innerhalb eines Restes untereinander ausgetauscht werden. | | |

### 2.Beispiel:

### Synthese von Gal(β1-4)GlaNAc(α1-UDP (UDP-LacNAc) mit β1-4Galactosyltransferase (EC 2.4.1.38) aus Rindermilch

Zur präparativen Synthese wurden 50 µmol (30.5 mg) UDP-Gal, 50 µmol (32.6 mg) UDP-GlcNAc (beides als Na₂-Salze von Sigma, Deisenhofen), 10 mg BSA, 2 U β1-4Galactosyltransferase aus Rindermilch (Sigma, München) in 5 ml 200 mM HEPES-NaOH pH 7.0 mit 1 mM MnCl₂ gelöst und unter sterilen Bedingungen inkubiert. Nach einer Inkubationszeit von 3 Tagen bei 30°C wurde die Reaktion durch ein kurzzeitiges Erhitzen (5 min) auf 95°C abgestoppt und mittels Ionenpaar-Reverse-Phase-HPLC Elling und Kula 1993 J. Biotechnology 29, 277-286 analysiert. Die Syntheseausbeute für UDP-LacNAc war 14.1% (bezogen auf den Akzeptor).

Die Produktisolierung erfolgte in 2 Schritten:
1) Abtrennung vorhandener Proteine durch Ultrafiltration in einer 10 ml Ultrafiltrationszelle von Amicon (Witten) mit einer YM 10-Membran (Ausschlußgröße: 10.000 Da).
2) Das Produkt UDP-LacNAc wurde durch wiederholte Gelfiltration an Biogel P2 (siehe Beispiel 1) bei 4°C von den übrigen Bestandteilen des Reaktionsgemisches abgetrennt und abschließend gefriergetrocknet.
Insgesamt wurde ca. 1 mg UDP-LacNAc isoliert.

### 3. Beispiel:

### Synthese von Gal(β1-4)GlcNAc(α1-UDP (UDP-LacNAc) mit β1-4Galactosyltransferasen (EC 2.4.1.38) aus verschiedenen Enzymquellen

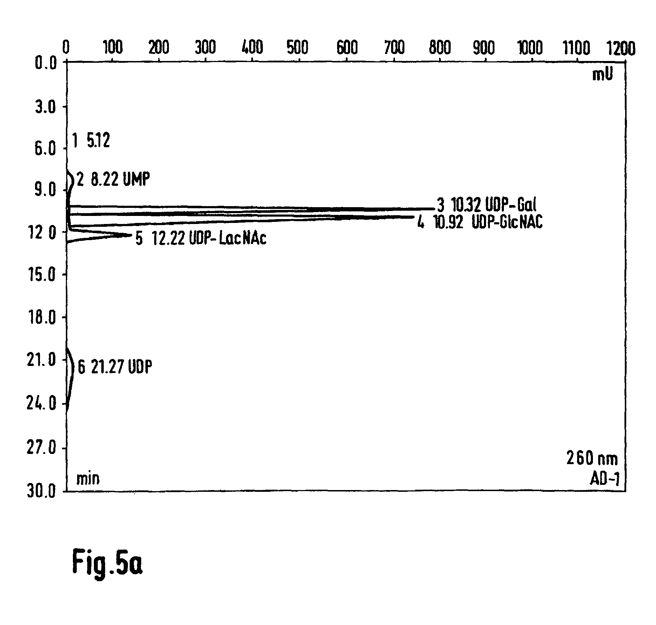

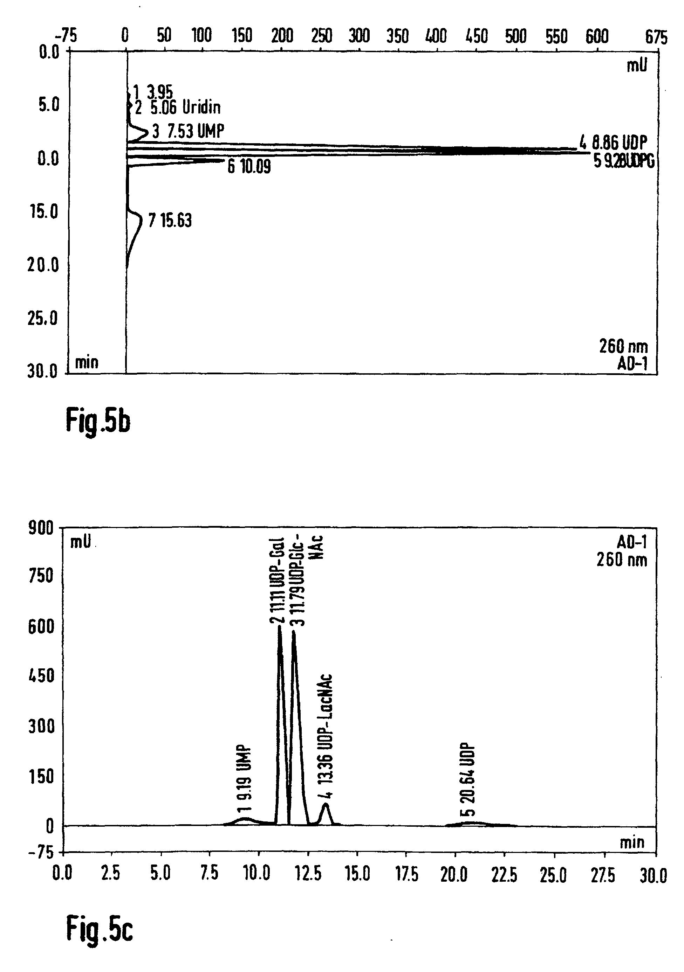

Unter optimierten Synthesebedingungen (10 mM UDP-Gal, 10 mM UDP-GlcNAc, 1 mg/ml BSA, 1 mM MnCl₂ in 200 mM Hepes-NaOH pH 7.0) wurden jeweils 0.4 U/ml β1-4Galactosyltransferase aus verschiedenen Enzymquellen eingesetzt und bei 30°C inkubiert. Dabei beziehen sich die Aktivitäten jeweils auf die Herstellerangaben. Nach der Inkubation bei 30°C wurde die Reaktion durch eine kurzzeitige Hitzedenaturierung der Enzyme (5 min. auf 95°C) abgestoppt und mittels Ionenpaar-Reverse-Phase-HPLC E1-ling und Kula 1993 J. Biotechnology 29, 277-286 analysiert. Die in der Figur 5 gezeigten HPLC-Chromatogramme verdeutlichen, daß bei allen Synthesen zwei Produkte UDP-LacNAc und UDP entstanden sind.

Fig. 5a) Galactosyltransferase aus Rindermilch 5.42 min. Uridin, 8.22 min. UMP, 10.32 min. UDP-Gal, 10,92 min. UDP-GlcNAc, 12.22 min. UDP-LacNAc, 21.27 min. UDP.

Fig 5b) Galactosyltransferase aus Humanmilch 5.06 min. Uridin, 7.53 min. UMP, 8.86 min. UDP-Gal, 9.28 min. UDP-GlcNAc, 10.09 min. UDP-LacNAc, 15.63 min. UDP. (Standard für 5a und 5c)

Fig.5c) Rekombinante, humane Galactosyltransferase 9.19 min. UMP, 11.11 min. UDP-Gal, 11.79 min. UDP-GlcNAc, 13.36 min. UDP-LacNAc, 20.64 min. UDP

Die Syntheseergebnisse sind in der Tabelle 2 zusammengefaßt.

**Tabelle 2:**

| Synthese von Gal(β1-4)GlcNAc(α1-UDP mit verschiedenen β1-4Galactosyltransferasen bei einer Inkubationstemperatur von 30°C. | | | |
|---|---|---|---|
| **β1-4Galactosyltransferase** | **Ausbeute (UDP- LacNAc) (%)** | **Inkubatuions- zeit (Tage)** | **Hersteller** |
| Rindermilch | 14.1 | 3 | Sigma |
| Humanmilch | 14.6 | 7 | Prof. Berger, ETH Zürich |
| rekombinante (humane) | 9.7 | 6 | Prof. Berger, ETH Zürich |

### 4. Beispiel:

### Tieftemperatursynthesen von Gal(β1-4)GlcNAc(α1-UDP mit β1-4Galactosyltransferasen (EC 2.4.1.38) aus verschiedenen Enzymquellen

Unter optimierten Synthesebedingungen (10 mM UDP-Gal, 10 mM UDP-GlcNAc, 1 mg/ml BSA, 1 mM MnCl₂ in 200 mM Hepes-NaOH pH 7.0) wurden jeweils 0.4 U/ml β1-4Galactosyltransferase aus verschiedenen Enzymquellen eingesetzt. Die Proben wurden 3 Minuten in flüssigem Stickstoff schockgefroren und dann bei -20°C inkubiert. Die Aktivität der β-Galactosyltransferase bezieht sich jeweils auf die Herstellerangaben. Nach der Inkubation bei -20°C wurde die Reaktion durch ein kurzzeitiges Erhitzen (5 min) auf 95°C abgestoppt und mittels Ionenpaar-Reverse-Phase-HPLC Elling und Kula 1993 J. Biotechnology 29, 277-286 analysiert. Die Ergebnisse in der Tabelle 3 zeigen, daß die Ausbeuten gegenüber den Versuchen bei 30°C (Beispiel 3) nach gleicher Inkubationszeit deutlich erhöht sind.

**Tabelle 3:**

| Tieftemperatursynthesen von Gal(β1-4)GlcNAc(1α-UDP mit verschiedenen β1-4Galactosyltransferasen. | | | |
|---|---|---|---|
| **β1-4Galactosyltransferase** | **Ausbeute (UDP- LacNAc) (%)** | **Inkubationszeit (Tage)** | **Hersteller** |
| Rindermilch | 20 | 7 | Sigma |
| Humanmilch | 16 | 7 | Prof. Berger, ETH Zürich |
| rekombinante (humane) | 19.4 | 6 | Prof. Berger, ETH Zürich |

### 5. Beispiel:

### Untersuchung des Substratspektrums der β1-4Galactosyltransferasen (EC 2.4.1.38) im Hinblick auf die Synthese weiterer aktivierter Disaccharide

10 mM UDP-Gal wurden mit jeweils 10 mM UDP-GlcNAc, 10 mM UDP-Xylose und 10 mM UDP-Glc in 200 mM Hepes-NaOH pH 7.0 mit 1 mg/ml BSA, 1 mM MnCl₂ und 0.4 U/ml β1-4GalT aus Humanmilch, Rindermilch bzw. rekombinante, humane GalT in Anwesenheit 0.1 und 8.0 mg/ml α-Lactalbumin bei 30°C inkubiert. Außerdem wurden 10 mM UDP-GalNAc mit jeweils 10 mM UDP-Glc, UDP-Gal und UDP-GlcNAc und zusätzlich jeweils 20 mM UDP-Glc, UDP-Xylose und UDP-Gal unter den gleichen Bedingungen inkubiert. Nach einer Inkubationszeit von 6 Tagen wurde die Reaktion durch ein kurzzeitiges Erhitzen (5 min.) auf 95°C abgestoppt und mittels Ionenpaar-Reverse-Phase-HPLC Elling und Kula 1993 J. Biotechnology 29, 277-286 analysiert. Mit der eingesetzten Analytik können entstehende Reaktionsprodukte wie UDP, UDP-LacNAc und UDP-Lactose nachgewiesen werden.

Die Ergebnisse sind in den Tabellen 4-6 zusammengefaßt.

**Tabelle 4:**

| Synthesen mit der β1-4Galactosyltransferase aus Humanmilch. | | | | |
|---|---|---|---|---|
| **Donor** | **Akzeptor** | **Ausbeute UDP (*ND*) [%]** | **Ausbeute UDP (*ND*) [%]** | **Ausbaute UDP (*ND*) [%]** |
| | | **α-Lactalbumin 0 mg/ml** | **α-Lactalbumin 1 mg/ml** | **α-Lactalbumin 8 mg/ml** |
| UDP-Gal | UDP-GalNAc | 1.2* *(0)* | 5.4* *(0)* | - |
| UDP-Gal | UDP-Glc | - | 4.8 *(0)* | - |
| UDP-GalN | UDP-Glc | 0 *(0)* | 0 *(0)* | - |
| UDP-GalNAc | UDP-GlcNAc | 0 *(0)* | 0 *(0)* | - |

| | | | | |
|---|---|---|---|---|
| * mit der gleichen Enzymcharge wurde 6 Monate früher eine Ausbeute von 3.0 % im Ansatz ohne α-Lactalbumin und 9.8 % UDP im Ansatz mit α-Lactalbumin erzielt. ***ND*: nucleotidaktiviertes Disaccharid** | | | | |

**Tabelle 5:**

| Synthesen mit der β1-4Galactosyltransferase aus Rindermilch. | | | | |
|---|---|---|---|---|
| **Donor** | **Akzeptor** | **Ausbeute UDP (*ND*) [%]** | **Ausbeute UDP (*ND*) [%]** | **Ausbeute UDP (*ND*) [%]** |
| | | **α-Lactalbumin 0 mg/ml** | **α-Lactalbumin 1 mg/ml** | **α-Lactalbumin 8 mg/ml** |
| UDP-Gal | UDP-Glc | 0.8 *(0)* | 2.0 *(0)* | 2.6 *(0)* |
| UDP-Gal | UDP-Xyl | 0 *(0)* | 2.9 *(0)* | 3.1 *(0)* |
| UDP-Gal | UDP-GlcNAc | 2.8 (*3.9*) | 4.0 (*1.2*) | 3.5 (*0*) |
| UDP-Gal | UDP-GalNAc | 0 *(0)* | 2.8 *(0)* | 3.4 *(0)* |
| UDP-GalNAc | UDP-Glc | 0 *(0)* | 0 *(0)* | 0 *(0)* |
| UDP-GalNAc | UDP-GlcNAc | 0 *(0)* | 0 *(0)* | 0 *(0)* |
| UDP-Glc | UDP-Glc | 0 *(0)* | 0 *(0)* | 0 *(0)* |
| UDP-Xyl | UDP-Xyl | 0 *(0)* | 0 *(0)* | 0 *(0)* |
| UDP-Gal | UDP-Gal | 0 *(0)* | 4.0 *(0)* | 3.8 *(0)* |

**Tabelle 6:**

| Synthesen mit rekombinanter, humaner β1-4Galactosyltransferase. | | | | |
|---|---|---|---|---|
| **Donor** | **Akzeptor** | **Ausbeute UDP (*ND*) [%]** | **Ausbeute UDP (*ND*) [%]** | **Ausbeute UDP (*ND*) [%]** |
| | | **α-Lactalbumin 0 mg/ml** | **α-Lactalbumin 1 mg/ml** | **α-Lactalbumin 8 mg/ml** |
| UDP-Gal | UDP-Glc | 6.4 *(0)* | 9.2 *(0)* | 6.6 *(0)* |
| UDP-Gal | UDP-Xyl | 0 *(0)* | 4.4 *(0)* | 4.1 *(0)* |
| UDP-Gal | UDP-GlcNAc | 4.5 (*4.7*) | 4.1 (*1.8*) | 3.8 *(0)* |
| UDP-Gal | UDP-GalNAc | 0 *(0)* | 1.2 *(0)* | 0.8 *(0)* |
| UDP-GalNAc | UDP-Glc | 0 *(0)* | 0 *(0)* | 0 *(0)* |
| UDP-GalNAc | UDP-GlcNAc | 0 *(0)* | 0 *(0)* | 0 *(0)* |
| UDP-Glc | UDP-Glc | 0 *(0)* | 0 *(0)* | 0 *(0)* |
| UDP-Xyl | UDP-Xyl | 0 *(0)* | 0 *(0)* | 0 *(0)* |
| UDP-Gal | UDP-Gal | 0 *(0)* | 1.6 *(0)* | 0 *(0)* |

Dieses Ergebnis zeigt, daß der Donor UDP-Gal nur auf den Akzeptor UDP-GlcNAc übertragen wird. Auffallend ist, daß die Bildung von UDP-LacNAc offensichtlich durch Anwesenheit von α-Lactalbumin zurückgedrängt wird. Bei der Kombination von UDP-Gal als Donor mit UDP-Glc als Akzeptor wird UDP, aber keine UDP-Lactose gebildet. Hier und bei der Kombination von UDP-Gal mit anderen UDP-Zuckern entsteht das UDP in Anwesenheit von α-Lactalbumin sehr wahrscheinlich durch eine enzymkatalysierte Hydrolyse von UDP-Gal (Übertragung von Galactose auf Wasser).

## Patentansprüche

1. Verfahren zur Herstellung von nucleotidaktivierten Sacchariden mit n Saccharid-Einheiten, wobei n = 2 bis oligo ist, bei dem ein Nucleosiddiphosphat-Zucker (NDP-Zucker) als Donor mit einem Nucleotidsaccharid mit n-1 Saccharid-Einheiten als Akzeptor enzymatisch umgesetzt wird.

2. Verfahren nach Anspruch 1,
**dadurch gekennzeichnet,**
**daß** die enzymatische Umsetzung mittels einer Glycosyltransferase erfolgt.

3. Verfahren nach Anspruch 2,
**dadurch gekennzeichnet,**
**daß** als Glycosyltransferase β1-4 Galactosyltransferase (EC 2.4.1.38) eingesetzt wird.

4. Verfahren nach Anspruch 2 oder 3,
**dadurch gekennzeichnet,**
**daß** die Glycosyltransferase eukaryontischen oder prokaryontischen Ursprungs ist.

5. Verfahren nach einem der Ansprüche 2 bis 4,
**dadurch gekennzeichnet,**
**daß** die Glycosyltransferase rekombinant ist.

6. Verfahren nach einem der Ansprüche 2 bis 5,
**dadurch gekennzeichnet,**
**daß** die Umsetzung bei einer Temperatur von -20°C erfolgt.

7. Verfahren nach einem der Ansprüche 1 bis 6,
**dadurch gekennzeichnet,**
**daß** das Nucleotidsaccharid mit n-1 Saccharid-Einheiten eine Komponente aus der Gruppe der Nucleotidsaccharide der Nucleotide von UDP, ADP, CDP, DTDP, GDP, CMP und IDP ist.

8. Verfahren nach einem der Ansprüche 1 bis 7,
**dadurch gekennzeichnet,**
**daß** ein Derivat eines Donor-NDP-Zuckers eingesetzt wird.

9. Verfahren nach einem der Ansprüche 1 bis 8,
**dadurch gekennzeichnet,**
**daß** als Akzeptor- Nucleotidsaccharid ein Nucleotidmonosaccharid eingesetzt wird, bei dem das Monosaccharid α-glycosidisch gebunden ist.

10. Verfahren nach einem der Ansprüche 1 bis 9,
**dadurch gekennzeichnet,**
**daß** als Akzeptor- Nucleotidsaccharid ein Derivat des N-acetylglucosamins oder des Glucosamins eingesetzt wird.

11. Verwendung von nucleotidaktivierten Di- oder Oligosacchariden, hergestellt nach dem Verfahren gemäß einem der Ansprüche 1 bis 10, als Standard für die biochemische Analytik.

12. Verwendung von nucleotidaktivierten Di- oder Oligosacchariden als Donatorsubstrate für Leloir-Glycosyltransferasen.

## Claims

1. Process for producing nucleotide-activated saccharides with n saccharide units, whereby n = 2 to an oligo-figure, in which a nucleoside diphosphate sugar (NDP-sugar) is enzymically converted as donor with a nucleotide saccharide with n-1 saccharide units as acceptor.

2. Process in accordance with claim I,
**characterized in that**
the enzymatic conversion occurs by means of a glucosyl-transferase.

3. Process in accordance with claim 2,
β1-4 galactosyl-transferase (EC 2.4.1.38) is used as glycosyl-transferase.

4. Process in accordance with claim 2 or 3,
**characterized in that**
the glycosyl-transferase is originally eukaryotic or prokaryotic.

5. Process in accordance with claims 2 to 4,
**characterized in that**
the glucosyl-transferase is recombinant.

6. Process in accordance with one of claims 2 to 5,
**characterized in that**
the conversion occurs at a temperature of -20°C.

7. Process in accordance with one of claims 1 to 6,
**characterized in that**
the nucleotide saccharide with n-1 saccharide units is a component from the group of nucleotide saccharides of the nucleotides UDP, ADP, CDP, DTDP, GDP, CMP and IDP.

8. Process in accordance with one of claims 1 to 7,
**characterized in that**
a derivative of a donor NDP sugar is used.

9. Process in accordance with one of claims 1 to 8,
**characterized in that**
a nucleotide monosaccharide is used in which the monosaccharide is α-glucosidically bound.

10. Process in accordance with one of claims 1 to 9,
**characterized in that**
a derivative of the N-acetyl-glucosamine or the glucosamine is used as an acceptor.

11. Use of nucleotide-activated di- or oligo-saccharides, produced in accordance with one of claims 1 to 10, as a standard for biochemical analysis.

12. Use of nucleotide-activated di- or oligo-saccharides as donor substrates for Leloir glycosyl-transferases.

## Revendications

1. Procédé de fabrication de saccharides activés par des nucléotides, ayant n unités de saccharides, où n est = 2 à oligo, dans lequel un sucre de nucléoside-diphosphate (sucre NDP), comme donneur, est transformé de manière enzymatique avec un nucléotide-saccharide, ayant n-1 unités de saccharide, comme accepteur

2. Procédé selon la revendication 1,
**caractérisé en ce que** la transformation enzymatique s'effectue au moyen d'une glycosyl-transférase.

3. Procédé selon la revendication 2,
**caractérisé en ce que** de la β1-4 galactosyl-transférase (EC 2.4.1.38) est utilisée comme glycosyl-transférase.

4. Procédé selon la revendication 2 ou 3,
**caractérisé en ce que** la glycosyl-transférase est d'origine eucaryote ou protocaryote.

5. Procédé selon l'une des revendications 2 à 4,
**caractérisé en ce que** la glycosyl-transférase est recombinante.

6. Procédé selon l'une des revendications 2 à 5,
**caractérisé en ce que** la transformation s'effectue à une température de -20°C.

7. Procédé selon l'une des revendications 1 à 6,
**caractérisé en ce que** le nucléotide-saccharide ayant n-1 unités de saccharides est une composante du groupe des nucléotides-saccharides des nucléotides de UDP, ADP, CDP, DTDP, GDP, CMP et IDP.

8. Procédé selon l'une des revendications 1 à 7,
**caractérisé en ce qu'**un dérivat d'un sucre NDP donneur est utilisé.

9. Procédé selon l'une des revendications 1 à 8,
**caractérisé en ce que** l'on utilise comme nucléotide-saccharide accepteur un nucléotide-monosaccharide dans lequel le monosaccharide est à liaison α-glycoside.

10. Procédé selon l'une des revendications 1 à 9,
**caractérisé en ce que** l'on utilise comme nucléotide-saccharide accepteur un dérivat de la N-acétylglucosamine ou de la glucosamine.

11. Utilisation de di ou d'oligosaccharides activés par nucléotides, fabriqués par le procédé selon l'une des revendications 1 à 10, comme standard pour l'analyse biochimique.

12. Utilisation de di ou d'oligosaccharides activés par nucléotides comme substrats de donneur pour glycosyl-transférases de Leloir.
